# EUROPEAN PATENT APPLICATION

(11) **EP 4 505 966 A1**
(43) Date of publication of application: **12.02.2025**
(21) Application number: 22940208.6
(22) Date of filing: 28.04.2022
(51) Int. Cl.: A61B 34/35

(54) **REMOTE OPERATION SYSTEM**

(71) Applicant: RIVERFIELD Inc., 1070052 Tokyo (JP)
(72) Inventor: TADANO, Kotaro, Tokyo 160-0017 (JP)
(74) Representative: adares Patent- und Rechtsanwälte Reininger & Partner GmbB
(86) International application number: PCT/JP2022/019244
(87) International publication number: WO 2023/209932

(57) **Abstract**

A remote operation system (100) includes an operation device (10) that remotely operates a device that acts on an object (O), a microphone (54) that detects sound caused by contact of the object with the device, a tactile sensation providing unit (56) that is provided in the operation device (10) and gives a tactile sensation to an operator, and a computing unit (58) that calculates an operation associated with the tactile sensation given by the tactile sensation providing unit (56). The computing unit 58 calculates an operation on the basis of the sound.

## Description

### Technical Field

The present invention relates to a remote operation system.

### Background Art

In current medical settings, surgical robots (manipulators) aimed at reducing the burden on operators during operations have increasingly been used. For example, a minimally invasive computer-assisted teleoperated surgery system allowing an operator to control one or more master input devices to perform surgical procedures on a patient through movements of associated remotely operated tools has been devised (refer to Patent Literature 1).

When using such surgical robots, however, an operator needs to rely only on visual information to perform an operation. The operation may therefore be affected by the fact that the operator does not feel with his/her thumb and fingers that which otherwise would be felt. For example, the operator may press on a patient's organ without noticing that forceps have hit the organ. Operators therefore need to go through extensive training before using such surgical robots and perform an operation based on the training. To overcome such disadvantages, there have been demands for development of functions of giving force sensation and tactile sensation to operators.

For example, Patent Literature 2 teaches a manipulation system in which an automatic operation of a slave manipulator that follows a manual operation of a master manipulator is controlled and in which the slave manipulator is operated primarily under force control by a pneumatically-driven system. This manipulation system has functions for giving force sensation to the operator.

### Related Art List

### Patent Literature

Patent Literature 1: JP 2022-048173 A
Patent Literature 2: WO 2008/108289 A1

### Summary of Invention

### Technical Problem

For the force sensation given to the operator by the aforementioned manipulation system, however, a relatively large reaction force received from an object is required. Thus, a small bump or contact with an object is hardly reflected in the force sensation. It is therefore difficult to perceive the hardness or the state of a surface of an object as a force sensation.

The present invention has been made in view of the aforementioned circumstances, and an exemplary object thereof is to provide a novel technology for allowing an operator to perceive the state of a surface of an object touched by a device.

### Solution to problem

To solve the aforementioned problems, a remote operation system according to an aspect of the present invention includes: an operation device that remotely operates a device that acts on an object; a microphone that detects sound caused by contact of the object with the device; a tactile sensation providing unit that is provided in the operation device and gives a tactile sensation to an operator; and a computing unit that calculates an operation associated with the tactile sensation given by the tactile sensation providing unit. The computing unit calculates the operation on the basis of the sound.

According to this aspect, information on an object, which cannot be sufficiently conveyed as force sensation, can be conveyed as tactile sensation to the operator when operating with the operation device. Note that the tactile sensation may be, for example, a sensation that can convey information on the hardness and the state (irregularity, etc.) of a surface of an object when the object is touched. The object may be, for example, an object to be grasped or cut with a device or a part of a human body to be treated with a surgical tool in an operation.

The tactile sensation providing unit may include an actuator that is driven in accordance with the sound detected by the microphone. This configuration enables a sensation when the device has touched the object through remote operation using the operation device to be indirectly conveyed in a form of vibration to the operator.

The actuator may be a voice coil motor capable of vibrating at least within a range of 200 to 400 Hz. This enables an operator to perceive the hardness of the object depending on the frequency of the vibration.

The remote operation system may further include a surgical tool being the device that is used for a surgical operation. The microphone may be provided near a contact position at which the object and the surgical tool come in contact with each other. Thus, the sound caused by contact of the object and the forceps can be accurately detected.

The microphone is capable of collecting sound at least within a range of 50 to 1000 Hz. Thus, sound at frequencies that can be caused by contact of the object and the device can be collected.

The surgical tool may be forceps having a shaft. The microphone may be provided on a main unit that detachably holds the forceps, and detect sound propagating through the forceps.

The remote operation system may include a separator that separates the main unit in a dirty area from the forceps in a clean area. A propagation member that transmits sound more easily than air may be provided between the separator and the forceps.

The surgical tool may be forceps having a shaft. An adhesive for fixing a microphone to the shaft may be provided. The microphone may be covered with an adhesive so that a sound collector thereof is not exposed. As a result, external sound including ambient sound is hardly collected by the sound collector, and structure-borne sound propagating through the shaft can be preferentially collected.

Urethane rubber may be provided between the microphone and the shaft. This enables collection of structure-borne sound with high accuracy and shutting out external sound.

Note that any combination of the components described above, and any expression in the present invention converted to that for a method, a device, a system, and the like also remain as aspects of the present invention.

### Advantageous Effects of Invention

The present invention allows an operator to feel the state of a surface of an object touched by a device.

### Brief Description of Drawings

FIG. 1 is a diagram illustrating a schematic configuration of a remote operation system according to an embodiment.
FIG. 2 is a schematic enlarged diagram of a structure around a connection between a microphone and forceps.
FIG. 3 is a side view illustrating a schematic configuration of an operation device according to the embodiment.
FIG. 4 is a top view illustrating a schematic configuration of the operation device according to the embodiment.
FIG. 5 is a side view for explaining a schematic configuration of a gimbal included in the operation device according to the embodiment.
FIG. 6 is a top view for explaining a schematic configuration of the gimbal included in the operation device according to the embodiment.
FIG. 7 is a side view illustrating a schematic configuration of a slave device according to a modification.
FIG. 8 is an enlarged cross-sectional view of a region E (see FIG. 7) of the slave device according to the modification.
FIG. 9 is an enlarged cross-sectional view of the region E (see FIG. 7) of a slave device according to another modification.

### Description of Embodiments

The present invention will now be described on the basis of an embodiment with reference to the drawings. Components, members, and processes that are the same as or equivalent to each other illustrated in the drawings are represented by the same reference numerals, and redundant explanation will not be repeated where appropriate. The embodiment is not to limit the invention, but is an example, and any feature or any combination of features described in the embodiment is not necessarily essential to the invention.

A remote operation system according to the embodiment is used for operation of a master manipulator of a master-slave surgical assist robot. An example of the surgical assist robot is one configured to operate forceps used in endoscopic surgery. Note that the application of the remote operation system is not limited to surgical assist robots. For example, the remote operation system may be used as a system for remotely operating robots used in logistics factories or manufacturing facilities. In particular, the remote operation system is suitable for remotely performing a process requiring long hours of delicate work by using a robot.

Note that the term remote not only includes a case where the physical distance between an operator and an object being operated is large but also a case where a master device operated by an operator and a slave device being operated is mechanistically separated from each other. In the latter case, the operation is remote operation even if the master device and the slave device are located close to each other.

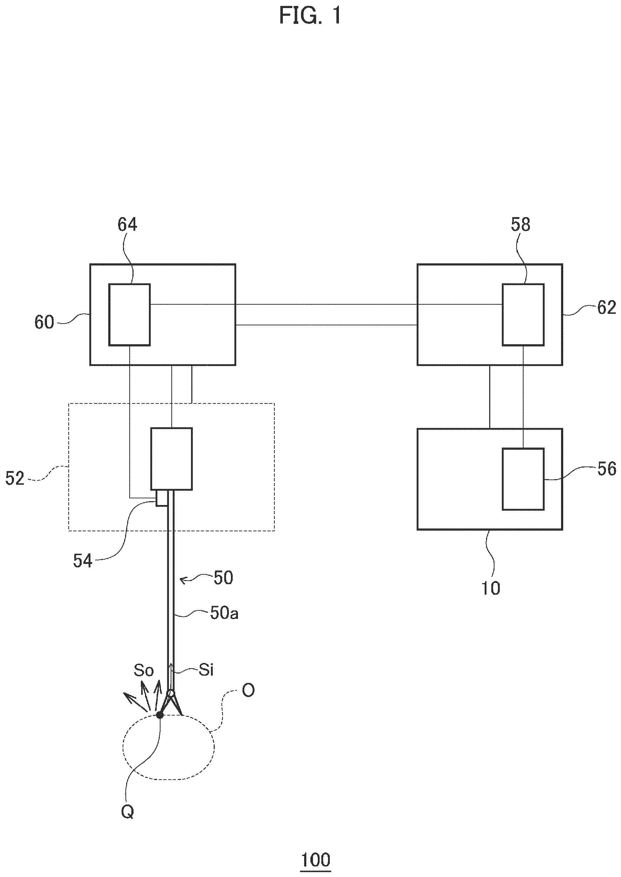

FIG. 1 is a diagram illustrating a schematic configuration of the remote operation system according to the embodiment. The remote operation system 100 includes an operation device 10, which is a master device for remotely operating a device acting on an object O, a slave device 52 including a mechanism for moving and driving forceps 50, which is the device, in accordance with the operation of the operation device 10, a microphone 54 that detects sound caused by contact of an object O with forceps 50, which is a surgical tool, a tactile sensation providing unit 56 that is provided in the operation device 10 and gives a tactile sensation to the operator, and a computing unit 58 that calculates an operation associated with the tactile sensation given by the tactile sensation providing unit 56.

The computing unit 58 calculates the operation on the basis of the sound detected by the microphone 54. The microphone 54 is located near a contact position Q at which the object O and the forceps 50 come into contact with each other. Thus, the sound caused by contact of the object O and the forceps 50 can be accurately detected.

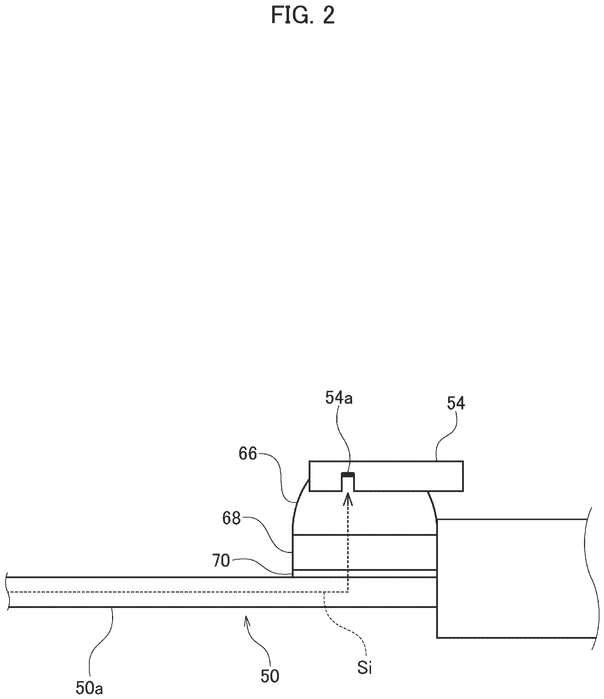

FIG. 2 is a schematic enlarged diagram of a structure around a connection between the microphone and the forceps. As illustrated in FIG. 1, the sound S caused when the object O and the forceps 50 come into contact with (hit) each other includes airborne sound So that is transmitted through the air outside the forceps 50 and structure-borne sound Si that propagates through a shaft 50a of the forceps 50.

The present inventor has conducted an intensive study on a desirable structure that can remove the airborne sound So, which is ambient sound, as much as possible and selectively collect the structure-borne sound Si that better reflects the attribute (such as the hardness and the surface state) of the object O that is hit. As illustrated in FIG. 2, the microphone 54 according to the embodiment is configured so that a sensor 54a, which is a sound collector, picks up the airborne sound So as little as possible, and is fixed to the outer circumference of a base portion of the shaft 50a of the forceps 50.

A recess in which the sensor 54a is located is preferably filled with a filling material so that the airborne sound So does not directly reach the sensor 54a. In the embodiment, an adhesive 66 is used as the filling material. In addition, a propagation member 68 for facilitating propagation of the structure-borne sound Si, which has traveled through the shaft 50a, to the sensor 54a is provided between the microphone 54 and the shaft 50a. The propagation member 68 and the microphone 54 are adhered to each other with the adhesive 66, and the propagation member 68 and the shaft 50a are adhered to each other with an adhesive 70.

For the microphone 54 according to the embodiment, a microelectromechanical system (MEMS) microphone (ICS-40180 manufactured by InvenSense, Inc.) that can be mounted on thin forceps is used. The frequency band of sound that can be collected by the MEMS microphone is 60 Hz to 20 kHz, and the supply voltage for the MEMS microphone is 1.5 to 3.6 V. The adhesive 66 and the adhesive 70 are hot-melt adhesives having thicknesses of about 0.5 to 1 mm. An example of alternative materials of the adhesives is epoxy resin. The propagation member 68 is urethane rubber with a hardness of A70 and a thickness of about 5±2 mm. The propagation member 68 may, however, be any material that better transmits sound than air. Examples of other materials include fluororubber. Furthermore, the materials and the forms of the adhesives are not particularly limited as long as the adhesives can make two members stick to each other. Alternatively, screws or springs may be used instead of the adhesives so that two members are pressed against each other and thus fixed.

In addition, the microphone 54 is located at the base portion of the shaft 50a, which is well away from the distal end of the forceps. This position is insusceptible to driving sound of wires inside the forceps caused by movement of an operating portion at the distal end of the forceps. Thus, the structure-borne sound Si can be accurately detected.

The remote operation system 100 further includes a slave controller 60 that controls a turning movement and a bending movement of individual components of the manipulator included in the slave device 52, and a master controller 62 that operates with the slave controller 60. The master controller 62 controls, in addition to the functions of the computing unit 58 described above, movements at the slave device 52 on the basis of information on the movements of individual components of the operation device 10. In addition, a signal processing unit 64 converts information on the sound detected by the microphone 54 for subsequent use by the computing unit 58. Specifically, the signal processing unit 64 performs filtering with a bandpass filter or noise attenuation using a dead band.

A signal processed by the signal processing unit 64 is supplied to the computing unit 58 in which a specific wavelength that affects the tactile sensation is enhanced, and then supplied to the tactile sensation providing unit 56. The tactile sensation providing unit 56 includes an actuator driven in accordance with the sound S detected by the microphone 54. This configuration enables conveyance of information on an object, which cannot be sufficiently conveyed as force sensation, as tactile sensation to the operator when operating with the operation device. Note that the tactile sensation is, for example, a sensation that can convey information on the hardness and the state (irregularity, etc.) of a surface of an object O when the object O is touched. The object O is, for example, an object to be grasped or cut with a device or a part of a human body to be treated with a surgical tool in an operation.

The actuator according to the embodiment is a voice coil motor capable of vibrating at least within a range of 200 to 400 Hz. Vibratory stimulation at a frequency in the range of 200 to 400 Hz is suitable for causing an operator to perceive the hardness of an object O. This configuration enables a sensation when the forceps 50 have touched the object O through remote operation using the operation device 10 to be indirectly conveyed in a form of vibration to the operator. This configuration also enables the operator to perceive the hardness of the object depending on the frequency of the vibration. Note that a piezoelectric element may be used as the actuator.

The microphone 54 according to the embodiment is capable of collecting sound at least within a range of 50 to 1000 Hz. This configuration enables collection of sound at a frequency correlated with the hardness, which can be generated when the object O and the forceps 50 come into contact with each other. Suitable examples of the microphone 54 include an MEMS microphone. Alternatively, a piezoelectric vibration sensor or an acceleration sensor may be used as a detector for detecting sound vibration. Still alternatively, a plurality of microphones and sensors may be combined to constitute a detector.

The mechanism of the operation device 10 according to the embodiment is not particularly limited but preferably includes joints of multiple degrees of freedom for use as a haptic device. Similarly, the mechanism of the slave device 52 preferably includes manipulator arms of multiple degrees of freedom for changing the position and the posture of the forceps 50. Furthermore, a pneumatic drive system capable of conveying force sensation may also be combined in the mechanism of the operation device 10 or the slave device 52.

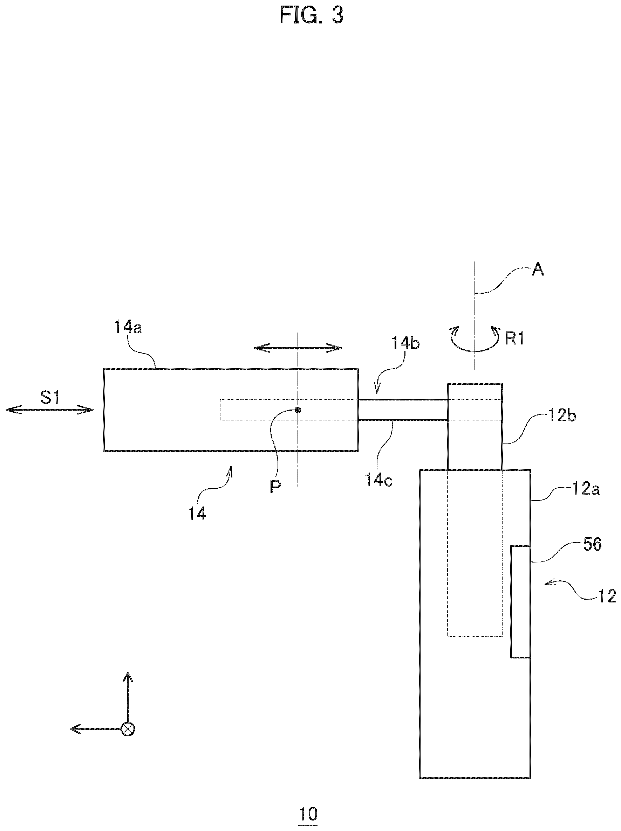

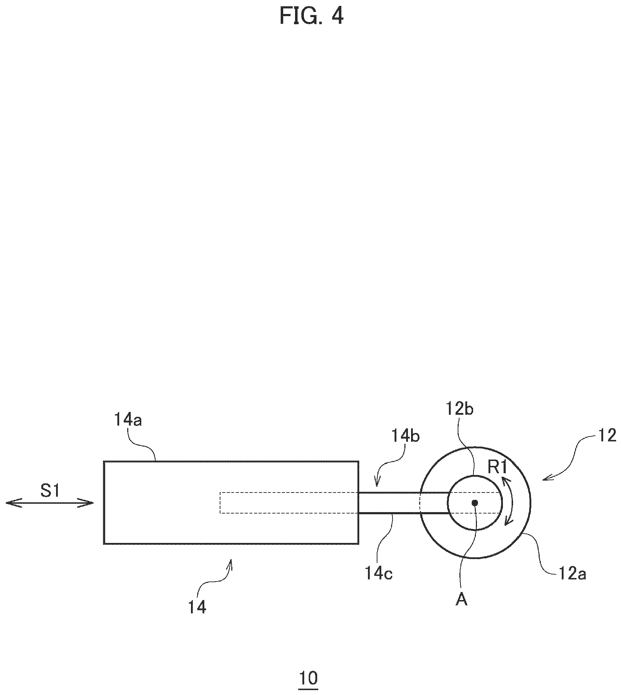

FIG. 3 is a side view illustrating a schematic configuration of the operation device according to the embodiment. FIG. 4 is a top view illustrating a schematic configuration of the operation device according to the embodiment. The operation device 10 is to be used for remote operation, and includes a grip portion 12 that is mainly held by the operator with a palm, and a pinch portion 14 that is mainly pinched by the operator with a thumb and fingers. The grip portion 12 includes an actuator (vibration generator) serving as the aforementioned tactile sensation providing unit 56 in an area to which the palm of the operator comes in contact. The pinch portion 14 is turnable (in the directions of arrows R1) relative to the grip portion 12 and slidable (in the directions of arrows S1) relative to the grip portion 12. Alternatively, the tactile sensation providing unit 56 may be provided at part of the pinch portion 14.

This configuration not only enables the whole operation device 10 to be moved with a power grip but also enables the pinch portion 14 to be turned and slid while the grip portion 12 is held by the palm. Thus, functions of both power grip operations and pinch grip operations can be provided, which reduces the burden on the operator's operation and enables delicate work.

The grip portion 12 includes a first hold 12a, having a cylindrical shape, to be directly held with a palm, and a turnable portion 12b that is turnable relative to the first hold 12a. The first hold 12a has a columnar hole in which the turnable portion 12b having a columnar shape is rotatably accommodated. The pinch portion 14 includes a second hold 14a to be directly held with a thumb and fingers, and a guide 14b that guides the second hold 14a to move away from and toward the grip portion 12.

This configuration enables the turnable portion 12b to turn in a state in which the first hold 12a of the grip portion 12 is fixed by the palm. In addition, the second hold 14a can be moved away from and toward the grip portion 12 with the thumb and fingers pinching the second hold 14a.

The turnable portion 12b is a rotating shaft provided in the first hold 12a. The guide 14b is connected to the rotating shaft, which is the turnable portion 12b, and fixed. In addition, the guide 14b includes a linear guide rail 14c extending in a direction intersecting the axis A of the rotating shaft. This enables the second hold 14a to be moved in predetermined directions (in the directions of arrows S1) relative to the grip portion 12. While the angle between the axis A and the extending direction of the guide rail 14c is 90° in the operation device 10 according to the embodiment, the angle may be other than 90° and may be set within a range of 90°±45°, within a range of 90°±30°, or within a range of 90°±15°.

Furthermore, the second hold 14a is guided by the guide rail 14c so as to move ±5 mm or more with respect to a reference point P relative to the rotating shaft. In other words, the second hold 14a is designed to be movable at least within a range of 10 mm along the guide rail 14c.

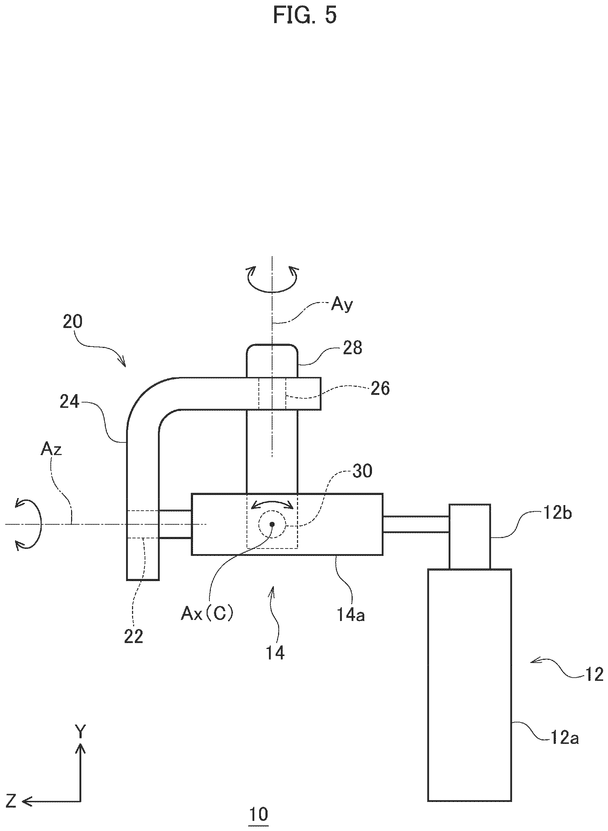

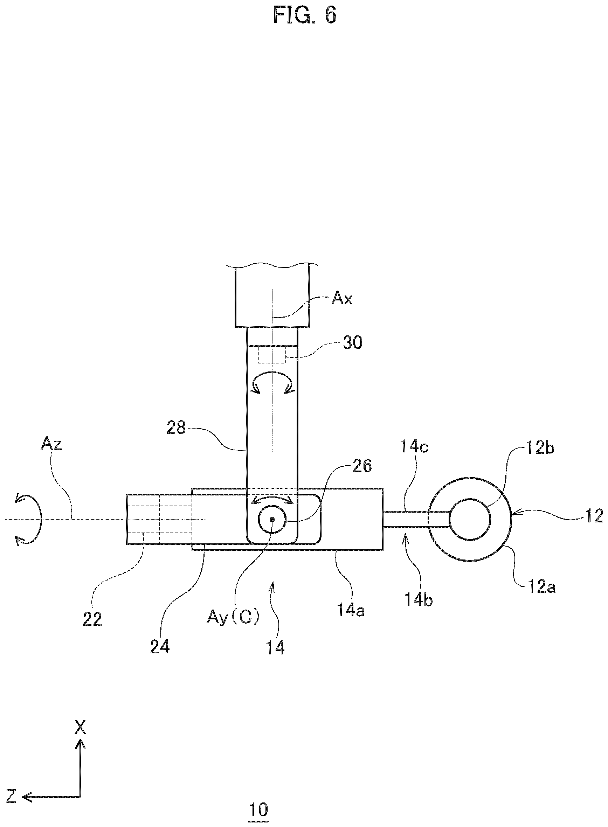

FIG. 5 is a side view for explaining a schematic configuration of a gimbal included in the operation device according to the embodiment. FIG. 6 is a top view for explaining a schematic configuration of the gimbal included in the operation device according to the embodiment.

The second hold 14a of the operation device 10 is movably supported by a gimbal 20. The gimbal 20 is turnable about three rotation axes that (orthogonally) intersect each other. The gimbal 20 is turnable about an X axis Ax, a Y axis Ay, and a Z axis Az.

Specifically, an end of the second hold 14a in the Z-axis direction is connected to a Z-axis portion 22, and the Z-axis portion 22 is rotatably held by a first arm 24 having an L shape. The first arm 24 also rotatably holds a Y-axis portion 26 and is rotatable relative to a second arm 28 connected with the Y-axis portion 26. The second arm 28 has an L shape, and rotatably holds an X-axis portion 30 at an end opposite the end at which the Y-axis portion 26 is held. The X-axis portion 30 is connected to a translation mechanism such as an XYZ stage or an XYZ link mechanism for movement in three-dimensional directions.

As a result, the movement information of the operation device 10 can be used for control of the position and posture of the manipulator of the slave device via the gimbal 20 and the translation mechanism. For example, in a case where the slave device is a surgical assist robot capable of manipulating forceps, the operator holds the grip portion 12 to move or turn the whole operation device 10 in a desired direction or to a desired orientation. The movement of the operation device 10 is detected by sensors (not illustrated) that obtain information (movement information) on the turning angles of the Z-axis portion 22, the Y-axis portion 26, and the X-axis portion 30. The movement information detected by the sensors is transmitted to the surgical assist robot. The surgical assist robot controls the movement of the position of the forceps on the basis of the transmitted movement information.

Note that the second hold 14a is slidable relative to the grip portion 12 with respect to a point (C in FIGS. 5 and 6) at which the three axes (the X axis Ax, the Y axis Ay, and the Z axis Az) of the gimbal 20 intersect each other. This can make the movement of a specific position of the manipulator of the slave device be associated with the sliding of the grip portion 12.

The remote operation system 100 including the operation device 10 illustrated in FIGS. 3 to 6 produces the following advantageous effects:
(1) allowing an operator to perceive a hit (impact) and a state of a surface of an object;
(2) allowing an operator to perceive even a contact and a change thereof causing a force sensation with a magnitude of nearly zero (which would be undistinguishable from errors);
(3) increasing the accuracy of an operator's perception of the texture and the hardness of an object being operated by combining a force sensation conveying mechanism in addition to a tactile sensation conveying mechanism; and
(4) providing event information (an alarm or operation guidance) of a system in a form of tactile sensation without interfering with an operation or eyesight.

### [Modifications]

In the embodiment described above, a case where the microphone is attached to the shaft with another member therebetween has been described. In the following modification, a configuration in which a microphone is provided on a main unit of a slave device to/from which forceps are attachable/detachable will be described.

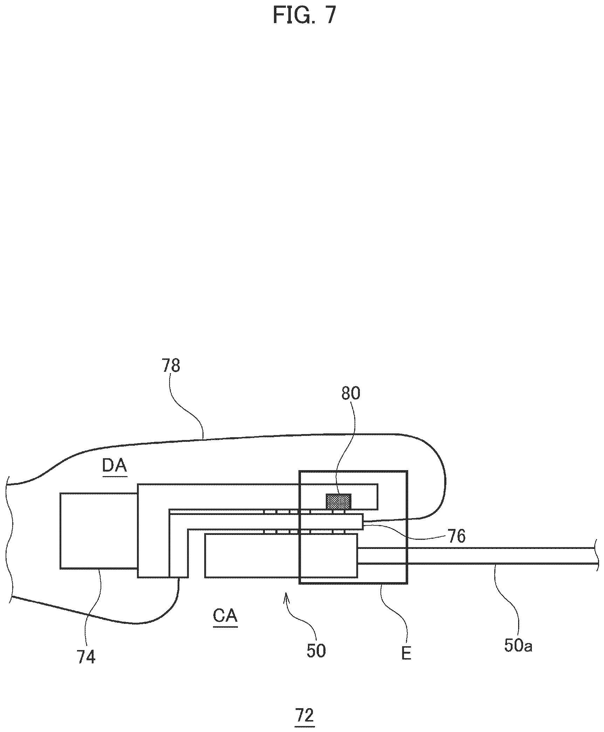

FIG. 7 is a side view illustrating a schematic configuration of a slave device according to a modification. A slave device 72 includes forceps 50 and a main unit 74 that detachably holds the forceps 50. A separator 76 that separates a dirty area DA from a clean area CA is provided between the main unit 74 and the forceps 50. A sterile drape 78 surrounds the dirty area DA on the main unit 74 side with respect to the separator 76. A microphone 80 is provided on a part of the main unit 74, and detects sound propagating through the forceps 50 and the separator 76.

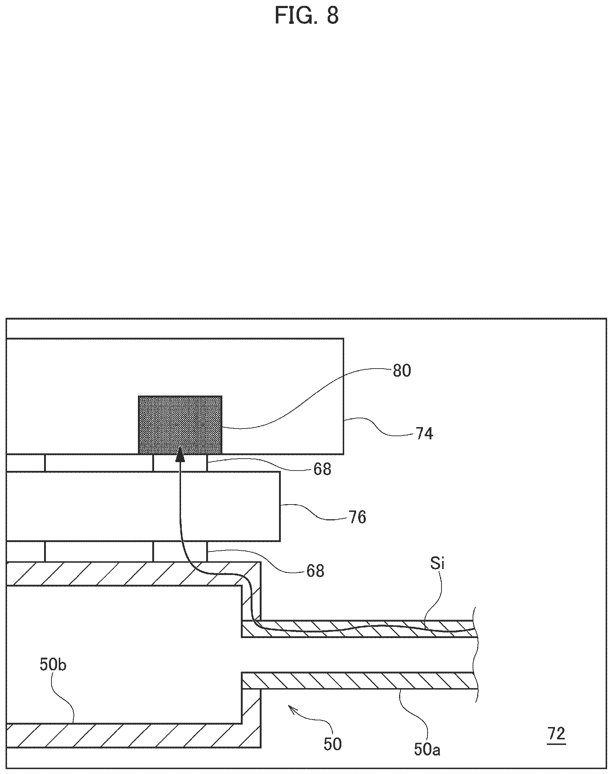

FIG. 8 is an enlarged cross-sectional view of a region E (see FIG. 7) of the slave device according to the modification. FIG. 8 illustrates a case where sound caused by the contact propagates as structure-borne sound Si through the forceps 50 and is detected by the microphone 80. The structure-borne sound Si propagates through solid portions of the shaft 50a and a housing 50b and reaches the microphone 80 via the propagation member 68 and the separator 76. This configuration allows even the microphone 80 in the dirty area DA to detect sound caused by contact between the forceps 50 and the object O.

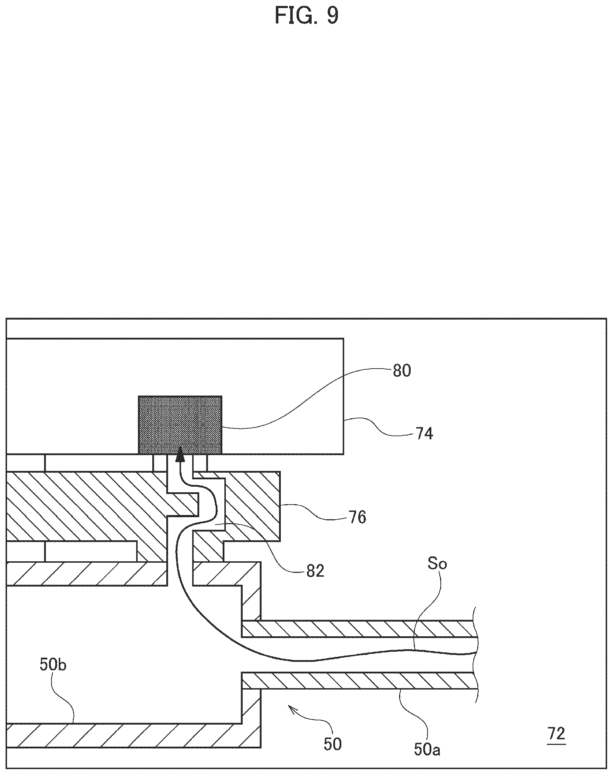

FIG. 9 is an enlarged cross-sectional view of the region E (see FIG. 7) of a slave device according to another modification. FIG. 9 illustrates a case where sound caused by the contact propagates as airborne sound So through the forceps 50 and is detected by the microphone 80. The airborne sound So propagates through internal spaces of the shaft 50a and the housing 50b and reaches the microphone 80 via a through-passage 82 inside the separator 76. This configuration allows even the microphone 80 in the dirty area DA to detect sound caused by contact between the forceps 50 and the object O.

While the present invention has been described above with reference to an embodiment, the present invention is not limited to the embodiment, and any combination or substitution of components in the embodiment as appropriate is included in the present invention. In addition, modifications such as combinations, changes in the order of processes, and various changes in design in the embodiment may be made on the embodiment on the basis of knowledge of a person skilled in the art, and such modified embodiments may be within the scope of the present invention.

### Reference Signs List

- 10: operation device
- 12: grip portion
- 14: pinch portion
- 50: forceps
- 52: slave device
- 54: microphone
- 56: tactile sensation providing unit
- 58: computing unit
- 60: slave controller
- 62: master controller
- 64: signal processing unit
- 66: adhesive
- 68: propagation member
- 70: adhesive
- 100: remote operation system

### Industrial Applicability

The present invention can be applied to a remote operation system for assembly or sorting of products, surgery, or the like.

## Claims

1. A remote operation system comprising:
an operation device that remotely operates a device that acts on an object;
a microphone that detects sound caused by contact of the object with the device;
a tactile sensation providing unit that is provided in the operation device and gives a tactile sensation to an operator; and
a computing unit that calculates an operation associated with the tactile sensation given by the tactile sensation providing unit,
wherein the computing unit calculates the operation on the basis of the sound.

2. The remote operation system according to claim 1,
wherein the tactile sensation providing unit includes an actuator that is driven in accordance with the sound detected by the microphone.

3. The remote operation system according to claim 2,
wherein the actuator is a voice coil motor capable of vibrating at least within a range of 200 to 400 Hz.

4. The remote operation system according to any one of claims 1 to 3, further comprising:
a surgical tool being the device that is used for a surgical operation,
wherein the microphone is provided near a contact position at which the object and the surgical tool come in contact with each other.

5. The remote operation system according to claim 4,
wherein the microphone is capable of collecting sound at least within a range of 50 to 1000 Hz.

6. The remote operation system according to claim 4,
wherein the surgical tool is forceps having a shaft, and
wherein the microphone is provided on a main unit that detachably holds the forceps, and detects sound propagating through the forceps.

7. The remote operation system according to claim 6, further comprising:
a separator that separates the main unit in a dirty area from the forceps in a clean area,
wherein a propagation member that transmits sound more easily than air is provided between the separator and the forceps.
